# NEW EUROPEAN PATENT SPECIFICATION

(11) **EP 1 919 402 B2**
(45) Date of publication and mention of the opposition decision: **29.07.2015**
(45) Mention of the grant of the patent: 11.11.2009
(21) Application number: 06745251.6
(22) Date of filing: 31.03.2006
(51) Int. Cl.: A61F 2/34

(54) **ENDOPROSTHESIS FOR ORTHOPEDIC APPLICATIONS**
ENDOPROTHESE FÜR ORTHOPÄDISCHE ANWENDUNGEN
ENDOPROTHÈSE POUR APPLICATIONS ORTHOPÉDIQUES

(43) Date of publication of application: 14.05.2008
(73) Proprietor: Sintea Biotech S.p.A., 20021 Baranzate (Milano) (IT)
(72) Inventor: SALA, Giuseppe, I-20033 Desio (Milano) (IT); GUERRA, Paolo, I-20145 Milano (IT)
(74) Representative: Postiglione, Ferruccio
(86) International application number: PCT/IT2006/000206
(87) International publication number: WO 2007/113862

(56) References cited:
- EP-A- 0 235 606
- EP-A- 0 329 019
- EP-A- 0 420 542
- EP-A- 0 420 795
- DE-A1- 3 310 944
- US-A- 4 959 072
- US-A- 5 728 510
- US-A1- 2002 173 854

## Description

The present invention relates to endoprostheses for orthopaedic applications; in particular, the present invention relates to a prosthesis for cotyloid cavity, suitable for seating the head of a femur for ensuring the functionality of the coxofemoral articulation.

Endoprostheses for orthopaedic applications have the function of replacing or cooperating with the components of the skeletal system in their main function, of standing mechanical stresses. In both cases (of replacement or "collaboration"), endoprostheses must as soon as better as possible "integrate" with the newly formed bony tissue, that is, cause the so-called arthrodesis, that is, the fusion between endoprosthesis and bone. In the art, such object is for example achieved by the use of osteo-conductive materials, which allow the taking root and proliferation of the bony tissue. The best solutions are represented by titanium, or by titanium alloys with aluminium-vanadium or niobium-zirconium.

The use of osteo-inductive coatings that stimulate the growth and proliferation of the bony tissue is also known in the art. The best solutions are represented by bioglasses and hydroxy-apatite.

The use of surfaces having morphologies suitable for the incorporation and/or mechanical grip of the bony tissue is also known. The best solutions adopted so far consist in making rough surfaces, obtained by "plasma pore" techniques or by sintering metal micro-balls.

The above solutions of the prior art are not alternative, but they can be put into effect at the same time, thus making endoprostheses of osteo-conductive material, having rough surface coated with osteo-inductive coating.

If on the one side the selection of the osteo-conductive material and of the osteo-inductive coating has determined "legitimate" solutions, the excellent method for obtaining a sufficiently rough surface for favouring the bony tissue gripping has not been found yet.

In fact, all the techniques currently used imply the coating of the prosthesis body with a layer added at a later time through plasma or sintering techniques. This causes a discontinuity and thus weakness of the interface between substrate and coating. Moreover, such coatings are not capable of forming effective holds for the growing bone, and therefore bring to an effective steady and irreversible osteo-integration.

These problems characterise all endoprostheses for orthopaedic applications and in particular, those endoprostheses that in the normal use are subject to considerable stresses.

The coxofemoral articulation is very complex and is one of the most stressed articulations in the human body.

The cotyloid cavity prostheses of the prior art, comprising a spherical cap suitable for receiving the head of a femur and being fixed by screws to the seating of a cotyloid cavity, do not ensure a quick, steady and lasting gripping at the cotyloid cavity. Such prostheses are known from EP0329019. The preamble of claim 1 in based on document US 5108435.

A quick and complete osteo-integration is very important since the femur head, as seen, is stressed by considerable forces.

The quickness of the osteo-integration allows the patient to recover the use of the limb very quickly, avoiding or reducing long and difficult periods of rehabilitation consequent to prolonged stasis and immobility.

The problem of the present invention is to provide a prosthesis which should solve the disadvantages mentioned with reference to the prior art.

Such disadvantages are solved with a prosthesis in accordance with claim 1.

Other embodiments of the prosthesis according to the invention are described in the subsequent claims.

Further features and the advantages of the present invention will appear more clearly from the following description of preferred non-limiting embodiments thereof, wherein:

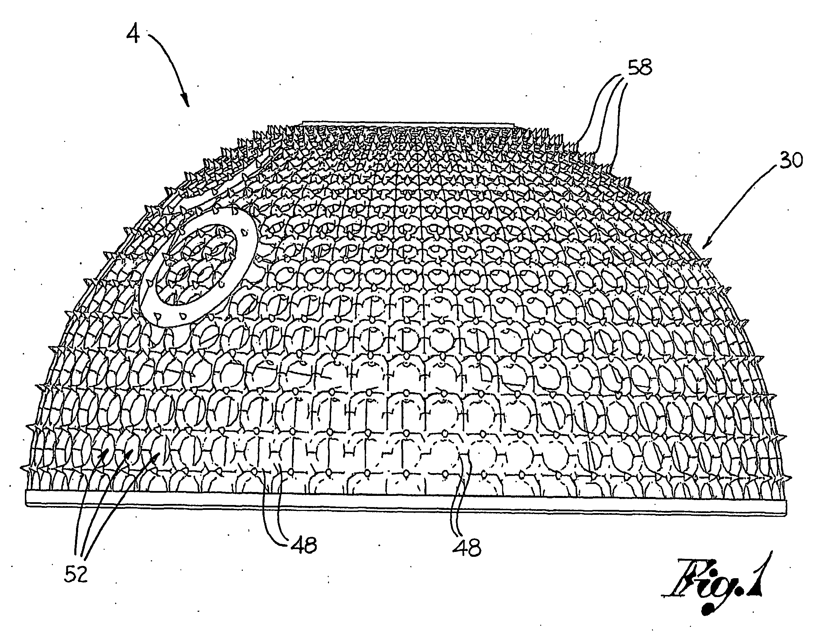

figure 1 shows a perspective view of a prosthesis for cotyloid cavity according to an embodiment of the present invention;

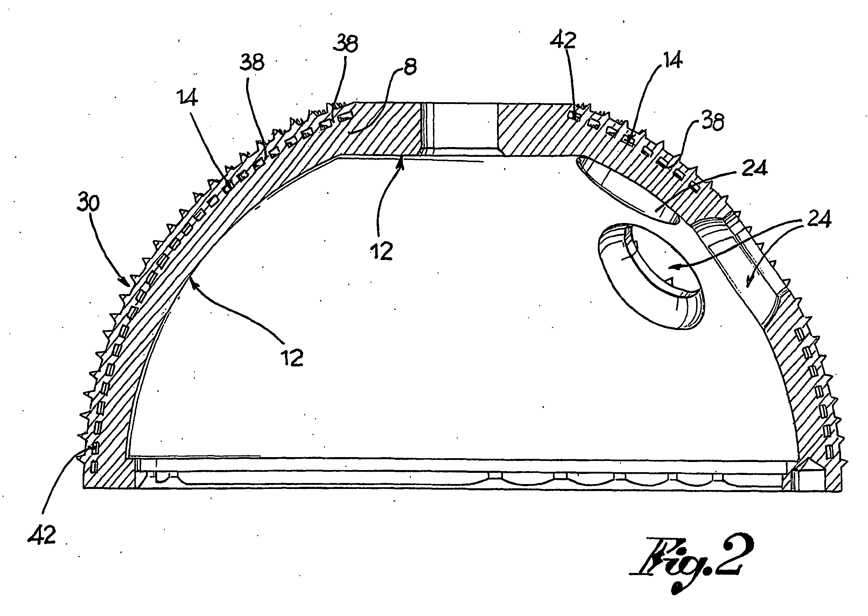

figure 2 shows a section view of the prosthesis of figure 1, along the section line II-II of figure 1;
figure 3 shows a perspective view of the prosthesis of figure 1 in a configuration where it is mounted on a femur head.

Elements or parts of elements in common between the embodiments described below are referred to with the same reference numerals.

With reference to the above figures, reference numeral 4 generically denotes an orthopaedic endoprosthesis.

According to an embodiment, prosthesis 4 comprises a prosthesis body 8 suitable for interfacing between a first and a second bone or portions of bone. The prosthesis body 8 has the function of providing the necessary mechanical stiffness to the connection between the first and the second bone, that is, of absorbing the forces exchanged between the bones.

The prosthesis body 8 is provided with a first wall 12 suitable for interfacing with the first bone and a second wall 14 suitable for interfacing with the second bone.

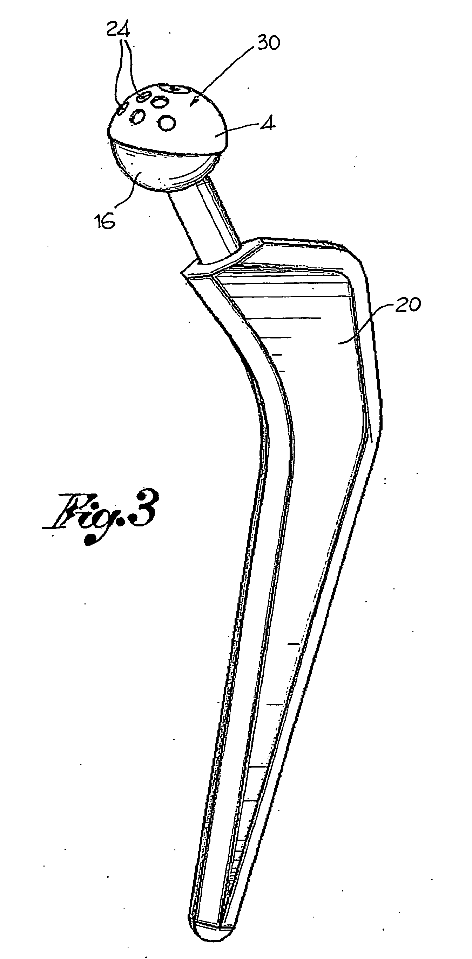

According to an advantageous embodiment (figure 3), prosthesis 4 is a prosthesis for cotyloid cavity, wherein the first bone is head 16 of a femur 20, whereas the second bone is a cotyloid cavity.

Prosthesis 4 comprises means for fixing the prosthesis body 8 suitable for obtaining a fixing of prosthesis 4 to at least one between the first and the second bone.

According to an embodiment, said fixing means comprise at least one connection hole 24 passing through the prosthesis body and at least one threaded connection means, such as a screw, suitable for passing through the connection holes 24 and for being screwed to the bone, for example in the cotyloid cavity.

The prosthesis body 8, at at least one of said walls 12, 14, comprises a fixing interface 30 suitable for favouring the osteo-integration with an associable bone; in other words, the fixing interface 30 has the function of favouring the bony growth so as to ensure the osteo-integration of the prosthesis.

The fixing interface 30 is spaced from one of walls 12, 14 of the prosthesis body 8 by a plurality of spacer elements 38 integrally connected to the prosthesis body 8 for forming a meatus 42 between interface 30 and the relevant wall 12, 14, said meatus 42 determining cavities and undercuts suitable for seating growing bony tissue.

The fixing interface is integrally associated to the prosthesis body; preferably, the fixing interface 30 is integral with the prosthesis body 8.

The fixing interface 30 comprises a reticular structure having a plurality of meshes 48 integrally interconnected to each other. Meshes 48 are stiffly connected to each other and thus, they cannot move or rotate relative to the prosthesis body 8.

Meshes 48 have a circular pattern, that is, they are comparable to discs, circular. Meshes 48 comprise a central hole 52 so as to take on a ring configuration.

Meshes 48 are rings having a circular section, relative to a section plane perpendicular to the associable wall 12, 14 of the prosthesis body 8. In other words, the meshes can be seen as rings formed by a 'wire' having circular section. According to further embodiments, the mesh section may be elliptical.

Preferably, meshes 48 are evenly distributed on the fixing interface 30, according to a regular and repetitive arrangement.

Meshes 48 may all have the same dimensions and shape, so as to form a homogeneous and even reticular structure on the entire interface 30. According to further embodiments of the present invention, the reticular structure may comprise meshes 48 having different dimensions.

Prosthesis 4, at each mesh 48 comprises at least one spacer element 38 integrally connected to the prosthesis body 8.

According to an embodiment, the spacer elements 38 are equal to each other and perpendicular to the relevant wall 12, 14 of the prosthesis body 8, so that meatus 42 between the prosthesis body and interface 30 is substantially constant. Moreover, interface 30 is counter-shaped relative to the profile of the underlying wall 12, 14 of the prosthesis body 8.

The spacer elements 38 are preferably perpendicular to walls 12, 14 of the prosthesis body 8 and have a column configuration. Preferably, the spacer elements have a circular section, but they may also have an elliptical, polygonal or quadrangular section. Moreover, the spacer elements may be cylindrical, that is, have a constant section along the extension thereof, or they may have a variable section, for example decreasing from wall 12, 14 towards meshes 48.

According to a further embodiment, the spacer elements 38 are different from each other, for example having different heights, so as to create variable meatus 42 between the prosthesis body 8 and the fixing interface 30.

Preferably, the fixing interface 30, on the side opposite to the prosthesis body 8, comprises a plurality of prickles 58 suitable for sticking at least partly into the associable bone for improving the primary stability of the prosthesis.

Said prickles 58 are for example arranged on the fixing interface 30 at the spacer elements 38 and opposite thereto relative to the interface. According to an embodiment, prickles 58 have a conical configuration for favouring the fixing to the bone. Preferably, prickles 58 have a height comprised between 0.1 mm and 1.5 mm, and even more preferably, said prickles 58 have a height comprised between 0.2 mm and 1.0 mm.

According to an advantageous embodiment of the present invention, prosthesis 4 is a prosthesis for cotyloid cavity having in all a spherical cap configuration, wherein the prosthesis body 8 is suitable for seating head 16 of a femur 20 at the first wall 12 according to a rotary-translatory coupling.

Prosthesis 4 for cotyloid cavity comprises, at the second wall 14, opposite the first wall 12, a fixing interface 30 shaped as a spherical cap, suitable for being associated to a cotyloid cavity.

The present invention is not limited to the use of prostheses for cotyloid cavity, but extends to all the possible types and shapes of endoprostheses whose purpose is to speed up and optimise the osteo-integration process, such as:
- in the field of major prosthetics: femoral cotyloid cavities and stems for hip bone prostheses, tibial plates, tibial and femoral stems for knee prostheses, glenoid components and humerus stems for shoulder prostheses;
- in the field of prosthetics for the vertebral column:
   intersomatic spacers, somatic cages, disk prostheses, vertebral plates, bars and screws;
- in the field of traumatology: synthesis plates for long bones, for hand and skull micro-surgery.

The above examples are not exhaustive and represent some of the possible advantageous uses of the present invention.

The present invention relates to endoprostheses made of different metals and metal alloys, such as (but not only): aluminium, copper, hafnium, lead, nickel, niobium, rhenium, stainless steels, tantalum, tin, titanium, tungsten, zinc, chromium, cobalt, molybdenum + all the possible combinations of these metals. At present, the most extensive and advantageous applications consist of titanium and alloys thereof, stainless steels, chromium-cobalt-molybdenum alloys, and nickel-chromium-cobalt-molybdenum alloys.

The prostheses may also be made of ceramics (at present alumina - aluminium oxide - and zirconia - zirconium oxide, but not only).

Moreover, the endoprostheses may also be made of polymers (at present PEEK - poly-ether-ether-ketone- and UHMWPE - high molecular weight polyethylene, but not only).

Preferably, meshes 48 exhibit a circular section having a diameter comprised between 0.1 mm and 0.5 mm; and even more preferably, said meshes 48 exhibit a circular section having a diameter equal to 0.2 mm.

Preferably, meshes 48 exhibit a circular ring pattern having a diameter comprised between 0.5 mm and 2 mm; even more preferably, said meshes 48 exhibit a circular ring pattern having a diameter equal to 1.0 mm.

Preferably, meatus 42 is comprised between 0.05 mm and 1 mm; and even more preferably, said meatus 42 is comprised between 0.1 mm and 0.5 mm.

It has been found that the above dimensional values ensure the best results in terms of bio-mimesis and osteo-integration.

A possible method of manufacture of a prosthesis according to the present invention shall now be described.

The technology for manufacturing the prostheses in accordance with the present invention belongs to the category of FFF (Free Form Fabrication) techniques. In particular, the technique preferably used is named EBM (Electron Beam Meting). Conceptually, it follows the rapid prototyping techniques, from which it differs in that it produces "finished" components capable of performing the functional tasks they are intended for, absolutely irrespective of the level of morphological complication.

In brief, the shape of the item to be manufactured (as complex, difficult, connected or not connected as desired) described by a CAD file is "cut" into thin slices (the more complex the shape, the thinner the slices). Such slices are then divided on their plane into "blocks" (similar to the "finished elements"), which are smaller as the complexity of the shape increases.

The shape, divided into "slices" and "blocks" is the information provided to a numerical control machine (a sort of three-dimensional plotter), whose "tool" consists of an electron beam.

This electron beam is piloted in a vacuum chamber inside which subsequent layers (corresponding to the above "slices") of very fine material powder are prepared. For each "slice" or layer, the electron beam moves according to a path dictated by the division into "blocks".

The specific energy (that is, energy by surface unit) of the electron beam is so high as to cause the instant melting and the immediate re-consolidation of the material. Where the electron beam does not pass, the material remains in the form of powder and is then eliminated, so as to leave the spaces occupied thereby empty.

As an alternative, among the FFF techniques it is also possible to use the selective laser-sintering which, similarly to the EBM technology, consists in addressing a laser beam having a specific energy sufficient for causing the instant and local melting of the portion of powder material impinged thereby.

In particular, the EBM technique is specifically used for making prostheses of titanium alloy, while the selective laser-sintering technique is for example used for making prostheses of Ch-Co based alloys.

As it can be understood from the description, the prosthesis according to the present invention allows overcoming the disadvantages of the prostheses of the prior art.

In particular, the prosthesis according to the invention allows facilitating the osteo-integration process, thanks to the creation of a plurality of cavities, interspaces and undercuts suitable for favouring the bone gripping to the prosthesis.

The particular circular shape of the mesh elements further favours the bony growth.

Moreover, the shape of the mesh wires in turn having circular section further favours the bony growth and grip, since it imitates the structure of the bony trabeculas.

In particular, the structures with circular section of the prosthesis according to the invention are geometrically similar to the bony trabeculas and thus improve the bio-mimesis and the osteo-integration. In particular, it has been found that the use of meshes with circular section, relative to a plane perpendicular to the fixing interface, favours the bony growth.

The prostheses according to the present invention ensure reduced bony growth and prosthesis osteo-integration times, as this is covered and incorporated in the grown bone.

Thanks to the presence of undercuts, the prosthesis anchoring is firm and steady over time and ensures high resistance even in applications on especially stressed bones, as in the case of the coxofemoral articulation.

The prostheses according to the present invention are particularly resistant since they are of the monolithic type, that is, they comprise a solid and compact prosthesis body, preferably of metal, having the function of standing the mechanical stresses to which a mesh element is integrally associated with the function of creating resistant and lasting bonds with the bone, by undercuts that facilitate the incorporation of the prosthesis itself.

Advantageously, the presence of the prickles on the outer surface of the mesh element facilitates and improves the primary stability of the prosthesis.

A man skilled in the art can make several changes and adjustments to the prostheses described above in order to meet specific and incidental needs. For example, a man skilled in the art could use osteo-conductive coatings, for example based on bioglasses or hydroxy-apatite, for further stimulating the growth and proliferation of the bony tissue.

These and other variations fall within the scope of protection as defined by the following claims.

## Claims

1. Endoprosthesis (4) for orthopaedic applications comprising
- a prosthesis body (8) suitable for interfacing between a first and a second bone or portions of bone,
- the prosthesis body (8) being provided with a first wall (12) suitable for interfacing with said first bone and a second wall (14) suitable for interfacing with said second bone,
- means for fixing the prosthesis body (8) suitable for obtaining a fixing of the prosthesis (4) to at least one between the first and the second bone,
- the prosthesis body (8), at at least one of said walls (12, 14) comprising a fixing interface (30) suitable for favouring the osteo-integration with an associable bone,
- the fixing interface (30) is spaced from one of the walls (12, 14) of the prosthesis body (8) by a plurality of spacer elements (38) integrally connected to the prosthesis body (8) for forming a meatus (42) between the interface (130) and the wall (12, 14), said meatus (42) determining cavities and undercuts suitable for seating growing bony tissue,
the fixing interface (30) comprising a reticular structure having a plurality of meshes (48) integrally interconnected to each other,
wherein the meshes (48) have circular section or elliptical section relative to a section plane perpendicular to the associable wall (12, 14) of the prosthesis body (8), **characterised in that** the meshes (48) comprise a central hole (52) so as to take on a ring configuration,
wherein said endoprosthesis is realised by an electron beam melting techniques or a laser sintering technique.

2. An endoprosthesis (4) according to claim 1, wherein said fixing interface (30) is integrally associated to the prosthesis body (8).

3. An endoprosthesis (4) according to claim 1 or 2, wherein said fixing interface (30) is integral with the prostheses body (8).

4. An endoprosthesis (4) according to any one of the previous claims, wherein the meshes (48) are evenly distributed on the fixing Interface (30) according to a regular and repetitive arrangement.

5. An endoprosthesis (4) according to any one of the previous claims, wherein said prosthesis (4) comprises at least one spacer element (38) integrally connected to the prosthesis body (3) so as to ensure at least one meatus (42) between the prosthesis body (8) and the fixing interface (30).

6. An endoprosthesis (4) according to claim 5, wherein said prosthesis comprises a spacer element (38) at each mesh (48).

7. An endoprosthesis (4) according to claims 5 or 6, wherein said spacer elements (38) are equal to each ocher and perpendicular to the relevant wall (12, 14) of the prosthesis body (8), so that the meatus (42) between the prosthesis body and the interface (30) is substantially constant.

8. An endoprosthesis (4) according to claim 7, wherein the interface (30) is counter-shaped relative to the profile of the underlying wall (12, 14) of the prosthesis body (8).

9. An endoprosthesis (4) according to claim 5 or 6, wherein said spacer elements (38) are different, so as to determine variable meatus (42) between the prosthesis body (8) and the interface.

10. An endoprosthesis (4) According to any one of the previous claims, wherein said fixing means comprise a plurality of connection holes (24) passing through the prosthesis body (8) and suitable for seating screw connection means for fixing the prosthesis to a bone.

11. An endoprosthesis (4) according to any one of the previous claims, wherein the fixing interface (30), on the side opposite to the prosthesis body (8), comprises a plurality of prickles (58) suitable for sticking at least partly into the associable bone for ensuring the primary stability of the prosthesis.

12. An endoprosthesis (4) according to claim 11, wherein said prickles (15) are arranged on the fixing interface (30) at the spacer element (38) and opposite thereto a relative to the fixing interface (30).

13. An endoprosthesis (A) according to claim 11 or 12, wherein said prickles have a conical configuration for favouring the fixing to the bone.

14. An endoprosthesis according to claims 11, 12 or 13, wherein said prickles (58) have a height comprised between 0.1 mm and 1.5 mm.

15. An endoprosthesis according to claims 14, wherein said prickles (58) have a height comprised between 0.2 mm and 1.0 mm.

16. An endoprosthesis according to any one of the previous claims, wherein the prosthesis (4) is a prosthesis for cotyloid cavity having in all a spherical cap configuration, wherein the prosthesis body (8) is suitable for seating the head (16) of a femur (20) at the first wall (12) according to a rotary-translatory coupling and on the second wall (14) exhibits a fixing interface (30).

17. An endoprosthesis according to claim 16, wherein the fixing interface (30) has a spherical cap pattern and is suitable for being associated to a cotyloid cavity.

18. An endoprosthesis according to any one of claims 1 to 17, made of metals and metal alloys belonging to a group comprising aluminium, copper, hafnium, lead, nickel, niobium, rhenium, stainless steels, tantalum, tin, titanium, tungsten, zinc, chromium, cobalt, molybdenum.

19. An endoprosthesis according to any one of claims 1 to 17, wherein the prosthesis is made of ceramic materials, such as alumina, aluminium oxide, zirconia, zirconium oxide.

20. An endoprosthesis according to any one of claims 1 to 17, wherein the endoprostheses are made of polymers, such as poly-ether-ether-ketone (PEEK) and/or high molecular weight polyethylene (UHMWPE).

21. An endoprosthesis According to any one of the previous claims, wherein an osteo-conductive coating is applied to said interface (30), on the side of the associable bone, for further stimulating the growth and the proliferation of the bony tissue.

22. An endoprosthesis according to claim 21, wherein said osteo-conductive coating comprises bioglasses and/or hydroxy-apatite.

23. An endoprosthesis according to any one of the previous claims, wherein said meshes (48) have a circular section having a diameter comprised between 0.1 mm and 0.5 mm.

24. An endoprosthesis according to claim 23, wherein said meshes (48) have a circular section having a diameter equal to 0.2 mm.

25. An endoprosthesis according to any one of the previous claims, wherein said meshes (48) exhibit a circular ring pattern having a diameter comprised between 0.5 mm and 2 mm.

26. An endoprosthesis according to claim 23, wherein said meshes (48) exhibit a circular ring pattern having a diameter equal to 1.0 mn.

27. An endoprosthesis according to any one of the previous claims, wherein said meatus (42) is comprised between 0.05 mm and 1 mm.

28. An endoprosthesis according to claim 27, wherein said meatus (42) is comprised between 0.1 mm and 0.5 mm.

29. A method of manufacture of an endoprosthesis according to any one of claims 1 to 28, said method comprising FFF (Free Form Fabrication) techniques, wherein said FFF technique is an EBM (Electron Beam Melting) technique comprising the steps of:
- arranging powder material on a support, into a vacuum chamber,
- addressing, according to the desired geometry, an electronic beam on the powder, said beam having a specific energy sufficient for causing the instant and local melting of a portion of powder material impinged thereby, so as to obtain the desired geometry,
- removing the powder not impinged by the beam,
wherein said FFF technique is a selective laser-sintering technique comprising the steps of:
- - arranging powder material on a support, into a vacuum chamber,
- - addressing, according to the desired geometry, a laser beam on the powder, said laser beam having a specific energy sufficient for causing the instant and local melting of a portion of powder material impinged thereby, so as to obtain the desired geometry,
- - removing the powder not impinged by the laser beam.

30. A method according to claim 29, wherein said ponder comprises a titanium alloy, when EBM technique is used.

31. A method according to claim 29, wherein said powder comprises a Ch-Co based alloy, when laser sintering technique is used.

## Patentansprüche

1. Endoprothese (4) für orthopädische Anwendungen, die umfasst:
- Einen Prothesenkörper (8), der geeignet ist, zwischen einem ersten und einem zweiten Knochen oder Teilen von Knochen zu koppeln,
- wobei der Prothesenkörper (8) mit einer ersten Wand (12), die geeignet ist, mit dem ersten Knochen zu koppeln, und einer zweiten Wand (14), die geeignet ist, mit dem zweiten Knochen zu koppeln, versehen ist,
- Mittel zum Befestigen des Prothesenkörpers (8), die geeignet sind, eine Befestigung bzw. Fixierung der Prothese (4) an wenigstens einem der ersten und zweiten Knochen dazwischen zu erreichen,
- wobei der Prothesenkörper (8) an wenigstens einer der Seitenwände (12, 14) eine Befestigungsgrenzfläche (30) umfasst, die geeignet ist, die Osteointegration mit einem verbindbaren Knochen zu begünstigen,
- wobei die Befestigungsgrenzfläche (30) von einer der Wände (12, 14) des Prothesenkörpers (8) durch eine Vielzahl von Abstandselementen (38), die integral mit dem Prothesenkörper (8) verbunden sind, beabstandet ist, um einen Gang bzw. Kanal (42) zwischen der Grenzfläche (30) und der Wand (12, 14) zu bilden, wobei der Gang (42) Kavitäten bzw. Hohlräume und Unterschneidungen bestimmt, die zum Anlagern von wachsendem Knochengewebe geeignet sind,
wobei die Befestigungsgrenzfläche (30) eine retikuläre bzw. netzförmige Struktur mit einer Vielzahl von integral miteinander verbundenen Maschen (48) umfasst, wobei die Maschen (48) einen kreisförmigen oder elliptischen Schnitt relative zu einer Schnittebene senkrecht zu der verbindbaren Wand (12, 14) des Prothesenkörpers (8) haben, **dadurch gekennzeichnet, dass** die Maschen (48) ein zentrales bzw. Mittleres Loch (52) umfassen, um einen Ringaufbau anzunehmen,
wobei die Endoprothese durch eine Elektronenstrahlschmelztechnik oder eine Lasersintertechnik realisiert wird.

2. Endoprothese (4) nach Anspruch 1, wobei die Befestigungsgrenzfläche (30) integral mit dem Prothesenkörper (8) verbunden ist.

3. Endoprothese (4) nach Anspruch 1 oder 2, wobei die Befestigungsgrenzfläche (30) integral mit dem Prothesenkörper (8) ist.

4. Endoprothese (4) nach einem der vorangehenden Ansprüche, wobei die Maschen (48) gemäß einer regelmäßigen und sich wiederholenden Anordnung gleichmäßig auf der Befestigungsoberfläche (30) verteilt sind.

5. Endoprothese (4) nach einem der vorangehenden Ansprüche, wobei die Prothese (4) wenigstens ein integral mit dem Prothesenkörper (8) verbundenes Abstandselement (38) umfasst, um wenigstens einen Gang (42) zwischen dem Prothesenkörper (8) und der Befestigungsgrenzfläche (30) sicherzustellen.

6. Endoprothese (4) nach Anspruch 5, wobei die Prothese ein Abstandselement (38) an jeder Masche (48) umfasst.

7. Endoprothese (4) nach den Ansprüchen 5 oder 6, wobei die Abstandselemente (38) untereinander gleich und senkrecht zu der relevanten Wand (12, 14) des Prothesenkörpers (8) sind, so dass der Gang (42) zwischen dem Prothesenkörper und der Grenzfläche (30) im Wesentlichen konstant ist.

8. Endoprothese (4) nach Anspruch 7, wobei die Grenzfläche (30) eine Gegenform relativ zu dem Profil der darunterliegenden Wand (12, 14) des Prothesenkörpers (8) ist.

9. Endoprothese (4) nach Anspruch 5 oder 6, wobei die Abstandselemente (38) unterschiedlich sind, um variable Gänge (42) zwischen dem Prothesenkörper (8) und der Grenzfläche zu bestimmen.

10. Endoprothese (4) nach einem der vorangehenden Ansprüche, wobei die Befestigungsmittel eine Vielzahl von durch den Prothesenkörper (8) gehenden Verbindungslöchern (24) umfassen, die geeignet sind, Schraubverbindungsmittel zum Befestigen der Prothese an einem Knochen zu lagern.

11. Endoprothese (4) nach einem der vorangehenden Ansprüche, wobei die Befestigungsgrenzfläche (30) auf der zu dem Prothesenkörper (8) entgegengesetzten Seite eine Vielzahl von Stacheln (58) umfasst, die geeignet sind, wenigstens teilweise in dem verbindbaren Knochen stecken zu bleiben, um die grundlegende bzw. Anfangsstabilität der Prothese sicherzustellen.

12. Endoprothese (4) nach Anspruch 11, wobei die Stacheln (15) auf der Befestigungsgrenzfläche (30) an den Abstandselementen (38) und relative zu der Befestigungsgrenzfläche (30) entgegengesetzt dazu angeordnet sind.

13. Endoprothese (4) nach Anspruch 11 oder 12, wobei die Stacheln einen konischen Aufbau haben, um die Befestigung an dem Knochen zu fördern.

14. Endoprothese nach den Ansprüchen 11, 12 oder 13, wobei die Stacheln (58) eine Höhe haben, die zwischen 0,1 mm und 1,5 mm enthalten ist.

15. Endoprothese nach Anspruch 14, wobei die Stacheln (58) eine Höhe haben, die zwischen 0,2 mm und 1,0 mm enthalten ist.

16. Endoprothese nach einem der vorangehenden Ansprüche, wobei die Prothese (4) eine Prothese für einen Gelenkpfannenhohlraum ist, der insgesamt einen kugelförmigen Kappenaufbau hat, wobei der Prothesenkörper (8) geeignet ist, um den Kopf (16) eines Oberschenkelknochens (20) gemäß einer drehendtranslatorischen Kopplung an der ersten Wand (12) zu lagern und auf der zweiten Wand (14) eine Befestigungsgrenzfläche (30) aufweist.

17. Endoprothese nach Anspruch 16, wobei die Befestigungsgrenzfläche (30) ein kugelförmiges Kappenmuster hat und geeignet ist, mit einem Gelenkpfannenhohlraum verbunden zu werden.

18. Endoprothese nach einem der Ansprüche 1 bis 17, die aus Metallen und Metalllegierungen gefertigt ist, welche zu einer Gruppe gehören, die Aluminium, Kupfer, Hafnium, Blei, Nickel, Niob, Rhenium, rostfreie Stahle, Tantal, Zinn, Titan, Wolfram, Zink, Chrom, Kobalt, Molybdän umfasst.

19. Endoprothese nach einem der Ansprüche 1 bis 17, wobei die Prothese aus keramischen Materialien, wie etwa Tonerde, Aluminiumoxid, Zirkonerde, Zirkonoxid, gefertigt ist.

20. Endoprothese nach einem der Ansprüche 1 bis 17, wobei die Endoprothesen aus Polymeren, wie etwa Poly-Ether-Ether-Keton (PEEK) und/oder ultrahochmolekulargewichtigem Polyethylen (UHMWPE), gefertigt sind.

21. Endoprothese nach einem der vorangehenden Ansprüche, wobei auf der Seite des verbindbaren Knochens eine osteoleitende Beschichtung auf die genannte Grenzfläche (30) aufgebracht ist, um das Wachstum und die Ausbreitung des Knochengewebes weiter zu stimulieren.

22. Endoprothese nach Anspruch 21, wobei die osteo-leitende Beschichtung Bioglase und/oder Hydroxyapatite aufweist.

23. Endoprothese nach einem der vorangehenden Ansprüche, wobei die Maschen (48) einen kreisförmigen Schnitt mit einem Durchmesser haben, der zwischen 0,1 mm und 0, 5 mm enthalten ist.

24. Endoprothese nach Anspruch 23, wobei die Maschen (48) einen kreisförmigen Schnitt mit einem Durchmesser gleich 0, 2 mm haben.

25. Endoprothese nach einem der vorangehenden Ansprüche, wobei die Maschen (48) ein kreisförmiges Ringmuster mit einem Durchmesser aufweisen, der zwischen 0,5 mm und 2 mm enthalten ist.

26. Endoprothese nach Anspruch 23, wobei die Maschen (48) ein kreisförmiges Muster mit einem Durchmesser gleich 1,0 mm aufweisen.

27. Endoprothese nach einem der vorangehenden Ansprüche, wobei der Gang (42) zwischen 0,05 mm und 1 mm enthalten ist.

28. Endoprothese nach Anspruch 27, wobei der Gang (42) zwischen 0,1 mm und 0,5 mm enthalten ist.

29. Verfahren zur Herstellung einer Endoprothese nach einem der Ansprüche 1 bis 28, wobei das Verfahren FFF-(Freiformfertigungs-) Techniken umfasst,
wobei die FFF-Technik eine EBM- (Elektronenstrahlschmelz) Technik ist, welche die folgenden Schritte umfasst:
- Anordnen von Pulvermaterial auf einer Halterung in einer Vakuumkammer,
- entsprechend der gewünschten Geometrie, Richten eines elektronischen Strahls auf das Pulver, wobei der Strahl eine spezifische Energie hat, die ausreicht, um das sofortige und lokale Schmelzen eines Teils des Pulvermaterials zu bewirken, auf das er auftrifft, um die gewünschte Geometrie zu erhalten,
- Entfernen des Pulvers, auf das der Strahl nicht aufgetroffen ist,
wobei die FFF-Technik eine selektive Lasersintertechnik ist, welche die folgenden Schritte umfasst:
- Anordnen von Pulvermaterial auf einer Halterung in einer Vakuumkammer,
- entsprechend der gewünschten Geometrie, Richten eines Laserstrahls auf das Pulver, wobei der Laserstrahl eine spezifische Energie hat, die ausreicht, um das sofortige und lokale Schmelzen eines Teils des Pulvermaterials zu bewirken, auf das er auftrifft, um die gewünschte Geometrie zu erhalten,
- Entfernen des Pulvers, auf das der Strahl nicht aufgetroffen ist.

30. Verfahren nach Anspruch 29, wobei das Pulver eine Titanlegierung umfasst, wenn EBM Technik verwendet ist.

31. Verfahren nach Anspruch 29, wobei das Pulver eine Ch-Co-basierte Legierung umfasst, wenn Lasersintertechnik verwendet ist.

## Revendications

1. Endoprothèse (4) pour applications orthopédiques comprenant
- un corps de prothèse (8) adapté pour assurer l'interface entre un premier et un second os ou des parties d'os,
- le corps de prothèse (8) étant doté d'une première paroi (12) adaptée pour assurer l'interface avec ledit premier os et d'une seconde paroi (14) adaptée pour assurer l'interface avec ledit second os,
- des moyens pour fixer le corps de prothèse (8) adaptés pour obtenir une fixation de la prothèse (4) à au moins un du premier et du second os,
- le corps de prothèse (8), au niveau d'au moins une desdites parois (12, 14) comprenant une interface de fixation (30) adaptée pour favoriser l'ostéo-intégration avec un os associable,
- l'interface de fixation (30) est espacée d'une des parois (12, 14) du corps de prothèse (8) par une pluralité d'éléments d'espacement (38) relies intégralement au corps de prothèse (8) pour former un méat (42) entre l'interface (30) et la paroi (12, 14), ledit méat (42) déterminant des cavités et découpes adaptées pour recevoir le tissue osseux en croissance, l'interface de fixation (30) comprenant une structure réticulaire ayant une pluralité de mailles (48) interconnectées intégralement les unes aux autres, dans laquelle les mailles (48) ont une section circulaire ou elliptique par rapport à un plan de coupe perpendiculaire à la paroi associable (12, 14) du corps de prothèse (8),
**caractérisée en ce que** les mailles (48) comprennent un orifice central (52) de sorte à prendre une configuration annulaire, dans laquelle ladite endoprothèse est réalisée au moyen d'une technique de fusion par faisceau d'électrons ou d'une technique de frittage au laser.

2. Endoprothèse (4) selon la revendication 1, dans laquelle ladite interface de fixation (30) est intégralement associée au corps de prothèse (8).

3. Endoprothèse (4) selon la revendication 1 ou 2, dans laquelle ladite interface de fixation (30) forme une seule pièce avec le corps de prothèse (8).

4. Endoprothèse (4) selon l'une quelconque des revendications précédentes, dans laquelle les mailles (48) sont réparties également sur l'interface de fixation (30) selon un agencement régulier et répétitif.

5. Endoprothèse (4) selon l'une quelconque des revendications précédentes, dans laquelle ladite prothèse (4) comprend au moins un élément d'espacement (38) relié intégralement au corps de prothèse (8) de sorte à assurer au moins un méat (42) entre le corps de prothèse (8) et l'interface de fixation (30).

6. Endoprothèse (4) selon la revendication 5, dans laquelle ladite prothèse comprend un élément d'espacement (38) au niveau de chaque maille (48).

7. Endoprothèse (4) selon les revendications 5 ou 6, dans laquelle lesdits éléments d'espacement (38) sont égaux et perpendiculaires à la paroi correspondante (12,14) du corps de prothèse (8), de sorte que le méat (42) entre le corps de prothèse et l'interface (30) est sensiblement constant.

8. Endoprothèse (4) selon la revendication 7, dans laquelle l'interface (30) a une forme contraire par rapport au profil de la paroi sous-jacente (12, 14) du corps de prothèse (8).

9. Endoprothèse (4) selon la revendication 5 ou 6, dans laquelle lesdits éléments d'espacement (38) sont différents, de sorte à déterminer un méat variable (42) entre le corps de prothèse (8) et l'interface.

10. Endoprothèse (4) selon l'une quelconque des revendications précédentes, dans laquelle lesdits moyens de fixation comprennent une pluralité d'orifices de liaison (24) passant à travers le corps de prothèse (8) et adaptés pour recevoir des moyens de vissage pour fixer la prothèse à un os.

11. Endoprothèse (4) selon l'une quelconque des revendications précédentes, dans laquelle l'interface de fixation (30), sur le côté opposé au corps de prothèse (8), comprend une pluralité d'épines (58) adaptées pour se fixer au moins en partie dans l'os associable pour assurer la stabilité principale de la prothèse.

12. Endoprothèse (4) selon la revendication 11, dans laquelle lesdites épines (15) sont agencées sur l'interface de fixation (30) au niveau des éléments d'espacement (38) et à l'opposé de ceux-ci par rapport à l'interface de fixation (30).

13. Endoprothèse (4) selon la revendication 11 ou 12, dans laquelle lesdites épines ont une configuration conique pour favoriser la fixation à l'os.

14. Endoprothèse selon les revendications 11, 12 ou 13, dans laquelle lesdites épines (58) ont une hauteur comprise entre 0,1 mm et 1,5 mm.

15. Endoprothèse selon la revendication 14, dans laquelle lesdites épines (58) ont une hauteur comprise entre 0,2 mm et 1,0 mm.

16. Endoprothèse selon l'une quelconque des revendications précédentes, dans laquelle la prothèse (4) est une prothèse pour cavité cotyloide ayant globalement une configuration de bouchon sphérique, dans laquelle le corps de prothèse (8) est adapté à recevoir la tête (16) d'un fémur (20) au niveau de la première paroi (12) selon un couplage par rotation translation et présente sur la seconde paroi (14) une interface de fixation (30).

17. Endoprothèse selon la revendication 16, dans laquelle l'interface de fixation (30) a une configuration de bouchon sphérique et est adaptée pour être associée à une cavité cotyloide.

18. Endoprothèse selon l'une quelconque des revendications 1 à 17, constituée de métaux et d'alliages de métaux appartenant à un groupe comprenant aluminium, cuivre, hafnium, plomb, nickel, niobium, rhénium, aciers inoxydables, tantale, étain, titane, tungstène, zinc, chrome, cobalt, molybdène.

19. Endoprothèse selon l'une quelconque des revendications 1 à 17, dans laquelle la prothèse est constituée de matériaux céramiques, tels que l'alumine, l'oxyde d'aluminium, la zircone, l'oxyde de zirconium.

20. Endoprothèse selon l'une quelconque des revendications 1 à 17, dans laquelle les endoprothèses sont constituées de polymères, tels que poly-éther-éther-cétone (PEEK) et/ou polyéthylène de masse moléculaire élevée (UHMWPE).

21. Endoprothèse selon l'une quelconque des revendication précédentes, dans laquelle un revêtement ostéo-conducteur est appliqué à ladite interface (30), sur le côté de l'os associable, pour stimuler en outre la croissance et la prolifération du tissu osseux.

22. Endoprothèse selon la revendication 21, dans laquelle ledit revêtement ostéo-conducteur comprend bioverres et/ou hydroxy-apatite.

23. Endoprothèse selon l'une quelconque des revendications précédentes, dans laquelle lesdites mailles (48) ont une section circulaire ayant un diamètre compris entre 0,1 mm et 0,5 mm.

24. Endoprothèse selon la revendication 23, dans laquelle lesdites mailles (48) ont une section circulaire ayant un diamètre égal à 0,2 mm.

25. Endoprothèse selon l'une quelconque des revendications précédentes, dans laquelle lesdites mailles (48) présentent une configuration d'anneau circulaire ayant un diamètre compris entre 0,5 mm et 2 mm.

26. Endoprothèse selon la revendication 23, dans laquelle lesdites mailles (48) présentent une configuration d'anneau circulaire ayant un diamètre égal à 1,0 mm.

27. Endoprothèse selon l'une quelconque des revendications précédentes, dans laquelle ledit méat (42) est compris entre 0, 05 mm et 1 mm.

28. Endoprothèse selon la revendication 27, dans laquelle ledit méat (42) est compris entre 0,1 mm et 0,5 mm.

29. Procédé de fabrication d'une endoprothèse selon l'une quelconque des revendications 1 à 28, ledit procédé comprenant des techniques de FFF (Free Form Fabrication - fabrication de forme libre),
dans laquelle ladite technique FFF est une technique EBM (Electron Beam Melting - fusion par faisceau d'électrons) comprenant les étapes consistant à :
- disposer ledit matériau en poudre sur un support, dans une chambre à vide,
- diriger, selon la géométrie souhaitée, un faisceau d'électrons sur la poudre, ledit faisceau ayant une énergie spécifique suffisante pour causer la fusion instantanée et locale d'une partie du matériau en poudre frappé par celui-ci, de sorte à obtenir la géométrie souhaitée,
- éliminer la poudre non frappée par le faisceau,
dans lequel ladite technique FFF est une technique de frittage au laser sélective comprenant les étapes consistant à:
- disposer ledit matériau en poudre sur un support, dans une chambre à vide,
- diriger, selon la géométrie souhaitée, un faisceau laser sur la poudre, ledit faisceau laser ayant une énergie spécifique suffisante pour causer la fusion instantanée et locale d'une partie du matériau en poudre frappé par celui-ci, de sorte à obtenir la géométrie souhaitée,
- éliminer la poudre non frappée par le faisceau laser.

30. Procédé selon la revendication 29, dans lequel ladite poudre comprend un alliage de titane, dans lequel la technique EBM est utilisée.

31. Procédé selon la revendication 29, dans lequel ladite poudre comprend un alliage à base de Ch-Co, dans lequel la technique de frittage au laser est utilisée.
